# EUROPEAN PATENT APPLICATION

(11) **EP 4 760 737 A1**
(43) Date of publication of application: **17.06.2026**
(21) Application number: 24220100.2
(22) Date of filing: 16.12.2024
(51) Int. Cl.: G16H 20/40, G16H 40/67, A61M 16/00

(54) **COMPUTER-IMPLEMENTED METHOD, COMPUTER PROGRAM PRODUCT, AND DEVICE RELATING TO USE OF A RESPIRATORY SUPPORT DEVICE**

(71) Applicant: Koninklijke Philips N.V., 5656 AE Eindhoven (NL)
(72) Inventor: DEN TEULING, Nicolaas Gregorius Petrus, Eindhoven (NL); GIL PONCE, Enrique Antonio, Eindhoven (NL)
(74) Representative: Philips Intellectual Property & Standards

(57) **Abstract**

There is provided a computer-implemented method comprising receiving use data representative of use of a respiratory support device by a subject. The method comprises determining, based on the use data, a feature value for each of a plurality of features. The plurality of features are human-interpretable features representative of use of the respiratory support device. The method comprises providing the feature values as input data to a machine learning model. The machine learning model is trained to generate predicted use data based on the feature values. The method comprises using the machine learning model to generate predicted use data representative of a predicted use of the respiratory support device by the subject, and determining an importance indicator for each of the plurality of features. The importance indicators are representative of a contribution of the corresponding feature to the predicted use. The method comprises generating an output signal indicative based on the importance indicators and the predicted use data. The output signal is representative of a likelihood the subject will stop using the respiratory support device.

## Description

### FIELD OF THE INVENTION

The invention relates to a computer-implemented method relating to use of a respiratory support device. The invention further relates to a computer program product and a device.

### BACKGROUND OF THE INVENTION

Sleep-disordered breathing (SDB), such as OSA and CSA, are becoming increasingly common, and are particularly prevalent in older people, people with a high body mass index, smokers, heavy drinkers and people with conditions such as coronary artery disease, hypertension, and diabetes mellitus. An untreated SDB may not only lead to daytime sleepiness due to interrupted sleep. An untreated SDB may cause or worsen serious health conditions, such as hypertension, high blood sugar, or cardiac problems.

To treat SDB, a subject with SDB is prescribed by a care professional to use a respiratory support device. The respiratory support device provides a pressurized airflow to the subject's airway. The pressurized airflow reduces or prevents collapse of the subject's upper airway during sleep. By reducing or preventing the collapse, the SDB is improved. By improving the SDB, the subject is less interrupted during sleep. This does not only improve daytime sleepiness, but may also prevent or reduce other health conditions.

Despite the health benefit of using the respiratory support device, many subjects do not adhere to using the respiratory support device as prescribed. Up to 50% of the subjects stop using the respiratory support device within one year of starting. Care professionals put in great effort to follow up on subjects in an attempt to keep the subjects using the respiratory support devices.

### SUMMARY OF THE INVENTION

Care professionals are not well able to identify subjects that are struggling with the respiratory support therapy and are likely to stop use. The group of subjects that struggles is large, whereas a subject may stop with the respiratory support therapy anywhere from a few weeks after the start of the respiratory support therapy till more than one year.

It is an objective of the invention to improve respiratory support therapy.

According to a first aspect, there is provided a computer-implemented method comprising receiving use data representative of use of a respiratory support device by a subject. The respiratory support device is adapted to provide a pressurized airflow to the subject during sleep. The method comprises determining, based on the use data, a feature value for each of a plurality of features. The plurality of features are human-interpretable features representative of use of the respiratory support device. The method comprises providing the feature values as input data to a machine learning model. The machine learning model is trained to generate predicted use data based on the feature values. The method comprises using the machine learning model to generate predicted use data representative of a predicted use of the respiratory support device by the subject. The method comprises determining an importance indicator for each of the plurality of features. The importance indicators are representative of a contribution of the corresponding feature to the predicted use. The method comprises generating an output signal indicative based on the importance indicators and the predicted use data. The output signal is representative of a likelihood the subject will stop using the respiratory support device.

In case the subject is struggling with the use of the respiratory support device, and is likely to stop use in the near future, the output signal is indicative of a high likelihood that the subject will stop using the respiratory support device. The high likelihood as indicated by the output signal, prompts the care professional to intervene before the subject stops using the respiratory support device. The features are human-interpretable features representative of use of the respiratory support device. This allows the care professional to understand which features have a high contribution to the predicted use. These features direct the care professional to help the subject to continue with using the respiratory support device.

For example, the feature indicates the subject uses the respiratory support only shortly at the beginning of night. This may represent that the subject is not able to fall asleep while using the respiratory support device. Based on the feature, the care professional adjusts the respiratory support device to reduce a ramp up of the pressure of the pressurized airflow. By reducing the ramp up, the pressurized airflow increases more slowly from a low pressure to a high pressure. The high pressure is the therapeutic pressure required by the subject. By reducing the ramp up, the subject is able to properly fall asleep while using the respiratory support device. As a result, the likelihood that the subject stops using the respiratory support device decreases.

For example, the feature indicates that the subject uses the respiratory support device for several hours, but then stops before the night is over. This may represent that the subject wakes up during the night and finds the patient interface too uncomfortable to continue use of the respiratory support device. The patient interface is coupled to the patient's face to provide the pressurized airflow to the subject's airway. The care professional may adjust the patient interface, or instruct the subject how to adjust the patient interface, such as adjusting straps that secure the patient interface to the subject's face. For example, the care professional may prescribe a different type of patient interface, such as a full face mask, an oral mask, a nasal mask, or a nasal pillow.

For example, the feature indicates that the subject uses the respiratory support device for several nights, but then stops use for several nights. This may represent that the subject has difficulties starting the respiratory support device. For some nights, the subject is able to put in enough time and effort to start the respiratory support therapy, whereas for other nights, the subject is not able to do so. The care professional may provide instructions to the subject on how to set up the respiratory support device and/or how to operate the respiratory support device.

Based on the output signal, the care professional may be directed to make any one of the following adjustments to the respiratory support device. In case the output signal indicates that the likelihood the subject will stop using the respiratory support device is high, the care professional may lower the pressure of the pressurized airflow. The lower pressure improves the comfort for the subject. As a result, the risk of the subject stopping use is reduced. Based on the output signal, the care professional may reduce the exhalation pressure of the pressurized airflow. Reducing the exhalation pressure allows the subject to exhale with less effort. The lower exhalation pressure improves the comfort for the subject. As a result, the risk of the subject stopping use is reduced. The inhalation pressure may be higher than the exhalation pressure to reduce the collapse of the upper airway of the subject.

So based on the output signal, the care professional is able to either adjust the respiratory support device, or to instruct the subject on how to use the respiratory support device. This improves the usage of respiratory support device by the subject. As a result, the respiratory support therapy is improved.

The use data is, for example, the use data of several nights or several weeks or several months. Experiments have shown that use data relating to only 3 nights of use is already sufficient to provide a reliable predicted use. Use data relating to 14 nights of use provides a large improvement of the reliability of the predicted use, with an estimated area under the curve (AUC) of about 0.94. The AUC is a well-known parameter to quantify the performance of a machine learning model. For example, the use data indicates whether the subject used the respiratory support device on a certain night or not. For example, the use data indicates how many hours per night the subject used the respiratory support device during a period of time.

The respiratory support device is, for example, a positive airway pressure (PAP) device. Examples of a PAP therapy device are a continuous PAP (CPAP) device, a bi-level PAP (BiPAP) device, or automatic PAP (AutoPAP) device. The CPAP device is adapted to provide pressurized air to the airway of the subject at a constant pressure level. The BiPAP device is adapted to provide pressurized air to the airway of the subject at an inhalation pressure level and an exhalation pressure level that is different from the inhalation pressure level. The AutoPAP device is adapted to provide pressurized air to the airway of the subject with a variable pressure within a pressure range.

The plurality of features are human-interpretable features representative of use of the respiratory support device. The values of the features are used as input data for the machine learning model. Machine learning models in general are able to use features that are human-interpretable and features that are not interpretable by humans. Because the output signal indicates the contribution of some or all of the features, the features used in the method are human-interpretable. This way, the care professional is able to understand the meaning of the features. For example, the features relate to periods of use or durations of use. For example, the features comprise one or more statistical features relating to the use of the respiratory support device. For example, the features comprise a mean time of use over multiple nights, or a minimum use over multiple nights, or a maximum use over multiple nights, or a standard deviation of the user over multiple nights. For example, the features comprise a statistical feature relating to a change in use over time, for example by using linear regression or a median regression approach.

The machine learning model is trained to generate predicted use data based on the feature values. For example, the machine learning model is trained according to the following method. Training use data is provided. The training use data is representative of use of respiratory support devices by a training population. The training population comprises a group of subjects. Each subject in the group uses a respiratory support device. The respiratory support devices are adapted to provide pressurized airflows to the subjects of the training population during sleep. The method comprises determining, based on the training use data, a feature value for each of the plurality of features. The plurality of features are the human-interpretable features representative of use of the respiratory support devices. The method comprises training the machine learning model to generate predicted use data based on the training use data and the feature values. The predicted use data is representative of a predicted use of the respiratory support device by a subject. The machine learning model is trained with the same features as used in the computer-implemented method. The use data for training the machine learning model may include use data extending over several months or years, such up to 2 years. This way, the way the subjects in the training population use the respiratory support devices is correlated to how long the subjects keep on using the respiratory support devices and how much the subjects keep on using the respiratory support devices. For example, the training of the machine learning model comprises using as input training data a first portion of the training use data corresponding to a first training time period, and using as output training data a second portion of the use data corresponding to a second training time period. The second training time period is after the first training time period. The machine learning model is trained to learn a relation between the input training data and the output training data. The machine learning model is trained to generate the predicted use data based on the relation.

The predicted use is, for example, an estimated time of use of the respiratory support device during a night in the future. For example, the night is one week, or two weeks, or one month, or 6 months in the future. The predicted use is, for example, an estimate of whether the time of use of the respiratory support device during a night in the future is more or less than a use threshold. For example, the use threshold is two hours, or four hours, or six hours per night. For example, the predicted use is an estimate of whether the subject will use or will not use the respiratory support device in the future. For example, the predicted use relates to whether the subject will meet in the future the CMS adherence reimbursement criterion as used in the United States. To meet this criterion the subject needs to use the respiratory support device in a 30 day period for at least 70% of the nights for at least four hours.

The importance indicators are representative of a contribution of the corresponding feature to the predicted use. The importance indicators indicate how important a feature is for the predicted use. Depending on the feature values, some features may contribute more to the predicted use than other features. For example, based on data from the machine learning model, the contribution of the features to the predicted use is determined. For example, the importance indicators are determined by making use of Shapley values. Shapley values provide a way to compute which features contributed to the predicted use and/or how much the features contributed to the predicted use. For example, the importance indicators are determined by making use of a method that assesses the impact of small perturbations of the feature values on the predicted use. For example, such a method uses Local Interpretable Model-Agnostic Explanations, "LIME". For example, the importance indicators are based on the contributions of the features to the predicted use as estimated over a period in the past, such as over the last 7 days, or the last 30 days. For example, the machine learning model comprises a logistic regression model. The importance indicators are based on model coefficients of the logistic regression model.

The likelihood is, for example, a percentage indicating the risk of the subject stopping in the near future. For example, 100% indicates it is almost certain the subject will stop in the near future, whereas 0% indicates the subject will almost certainly continue in the near future. For example, the likelihood has two values. One value indicates the risk of stopping is high, whereas the other value indicates the risk of stopping is low. For example, the likelihood has more values, such as three or four or five values, ranging from a low value to a high value. The low value is indicative of a low likelihood the subject will stop in the near future, whereas the high value is indicative of a high likelihood the subject will stop in the near future. For example, the likelihood that the subject will stop in the near future is high in case the predicted use data indicates the predicted use is lower than a present use. For example, the likelihood that the subject will stop in the near future is low in case the predicted use data indicates the predicted use is the same as the present use.

The output signal is, for example, adapted to cause a display to visualize the importance indicators and/or the likelihood, for example as an image or as text, or as a combination of an image and text. For example, the output signal is adapted to be transmitted to a computer of the care professional, for example via the internet or via blue tooth or via Wi-Fi or via other ways of telecommunication.

In an embodiment, the method comprises determining the likelihood the subject will stop using the respiratory support device based on the predicted use data.

In an embodiment, the use data comprises at least one of a duration of use, a start time of use, a stop time of use, a number of nights of use, a number of nights without use, or a number of consecutive nights without use.

In an embodiment, the plurality of features comprises at least one of a mean use of the respiratory support system per night, a number of nights without use, and a number of consecutive nights without use.

Experiments have shown that the mean use per night, and the number of nights without use provide a good basis for the prediction of the predicted use.

In an embodiment, the plurality of features comprises a statistical parameter based on the use data.

According to this embodiment, the features comprise a statistical parameter, such as a mean, a minimum, a maximum, and/or quantiles of the use. For example, the statistical parameter comprises a slope representing a change in the use. For example, the statistical parameter comprises the highest numbest consecutive nights without use. For example, the statistical parameter comprises a standard deviation, or a mean absolute difference (MAD), or a median absolute difference of the use data. For example, the statistical parameter is representative of a coefficient of robust variation, or a standardized maximum outlier.

In an embodiment, the computer-implemented method comprises retraining the machine learning model to learn a relation between input retraining data and output retraining data. The input retraining data comprises a first portion of the use data corresponding to a first use time period. The method comprises using as output retraining data a second portion of the use data corresponding to a second use time period. The second use time period is after the first use time period.

According to this embodiment, the machine learning model is retrained with the use data from the subject. When the subject uses the respiratory support device for a while, the use data contains sufficient information to correlate the use during the first use time period with the use during the second use time period. With this information, the machine learning model is retrained to more accurately provide the predicted use data for the subject.

In an embodiment, the first use time period is at least 90 days.

By using the information from the 90 days of use of the respiratory support device by the subject, the machine learning model can be retrained to provide the predicted use data with an increased accuracy.

In an embodiment, using the machine learning model comprises using an extreme gradient boosting (XGB) algorithm.

An XGB algorithm is able to learn interactions between many features. As a result, the machine learning model is able to make use of many features representative of use of the respiratory support device and to correlate those many features to the predicted use. As a result, an accurate predicted use is obtained.

In an embodiment, the computer-implemented method comprises comparing the importance indicators with at least one threshold. The threshold is representative of the importance indicator having at least a significant contribution to the predicted use. The method comprises determining a subset of importance indicators exceeding the at least one threshold. The method comprises generating the output signal based on the subset of importance indicators.

In an embodiment, the output signal is representative of a ranking of the importance indicators based on a contribution of the corresponding feature to the predicted use.

The output signal ranks the importance indicators based on their contribution to the predicted use. For example, the ranking has the importance indicators in descending order. This way the importance indicators of the features that contribute the most are on one side of the ranking, whereas the remaining features are on the other side of the ranking. This way, the care professional is directly guided towards the features that are contributing the most to the predicted use.

According to this embodiment, the output signal is indicative of the subset of importance indicators instead of all importance indicators. This way, only the importance indicators that significantly contribute to the predicted use are included in the output signal, whereas the importance indicators that have only a small or negligible contribution to the predicted use are excluded from the output signal. This reduces the amount of data required to generate the output signal. Also, it helps the care professional to focus on the largest contributors that cause the likelihood of the subject stopping use. For example, the subset has only 1 or 2 or 3 of the importance indicators. For example, the output signal is generated only if the likelihood the subject will stop use exceeds a threshold.

In an embodiment, the computer-implemented method comprises determining an odds ratio for at least one importance indicator. The odds ratio is representative of an association between the importance indicator and the predicted use. The odds ratio exceeds the at least one threshold when the odds ratio is outside a range of 90%-110%.

According to this embodiment, the odds ratio is used to quantify the association between the importance indicator and the predicted use. In case the odds ratio is around 100%, there is no association or only a very small association between the importance indicator and the predicted use. In case the odds ratio is lower than 90%, the odds ratio indicates that the importance indicator has a significant negative effect on the predicted use, i.e., an increase of the value of importance indicator reduces the predicted use. In case the odds ratio is higher than 110%, the odds ratio indicates that the importance indicator has a significant positive effect on the predicted use, i.e., an increase of the value of importance indicator increases the predicted use.

For example, in addition or alternative to the odds ratio, a relative risk or an absolute risk reduction is used to quantify the association between the importance indicator and the predicted use.

In an embodiment, the computer-implemented method comprises generating the output signal to include an alarm signal for alarming a care professional, in case the likelihood is above a critical threshold.

According to this embodiment, the alarm signal is generated when the likelihood is above the critical threshold. The critical threshold is indicative of a large or almost certain likelihood that the subject will stop using the respiratory support device. The alarm signal helps the care professional to distinguish the subject having the large likelihood from all the other subjects treated by the care professional. The alarm signal prompts the care professional to intervene, so it is prevented that the subject will stop using the respiratory support device.

In an embodiment, the output signal is indicative of one or more of i) a predicted time the subject continues using the respiratory support device, or ii) a predicted level of use at which the subject continues using the respiratory support device.

According to this embodiment, the output signal is indicative of one or more parameters relating to the predicted use. One of these parameters is the predicted time the subject continues using the respiratory support device. For example, the predicted time is per night or per day or per week. To have a health benefit from using the respiratory support device, the subject should use the respiratory support device at least a certain amount of time per night, such as at least 2 hours or at least 4 hours or at least 6 hours. Another of these parameters is the predicted level of use. For example, the predicted level of use represents how often the subject will skip using the respiratory support device or how many nights the subject will stop using the respiratory support device. By including these parameters, the output signal prompts the care professional to intervene.

In a second aspect of the invention, there is provided a computer program product, comprising instructions which, when executed by a processor cause the computer-implemented method according to the first aspect to be carried out.

In a third aspect of the invention, there is provided a device comprising a processor adapted to execute the computer program product of the second aspect.

In an embodiment, the device comprises a data receiver and a signal output. The data receiver is adapted to communicate with the respiratory support device to receive the use data from the respiratory support device. The processor is adapted to receive the use data from the data receiver. The signal output is adapted to communicate with a display device to provide the output signal to the display device.

In a fourth aspect of the invention, there is provided a computer-implemented method. The computer-implemented method comprises receiving for a plurality of subjects a plurality of output signals generated by the method according to the first aspect. The computer-implemented method comprises classifying the plurality of subjects to a plurality of classes based on the importance indicators and the likelihood. The computer-implemented method comprises selecting at least one of the plurality of classes as a candidate class for intervention. The computer-implemented method comprises generating a class output signal indicative of the importance indicators and the likelihood of the class for the candidate class for intervention.

According to the fourth aspect, the subjects in a class have similar importance indicators and likelihoods. One or more classes that have subjects that are highly likely to stop using the respiratory support device may be selected as the candidate class or the candidate classes for intervention. For a candidate class, the solution to reduce the likelihood is the same or similar for all the subjects in the class, because the importance indicators for the subjects in that class are similar. Based on the class output signal, the care professional is able to adjust the respiratory support device, or to instruct the subjects in the class how to use the respiratory support device to reduce the likelihood the subjects will stop use. For example, the care professional may reduce the pressure of the pressurized air provided by the respiratory support devices for one candidate class, whereas the care professional provides instruction to subjects in another candidate class how to attach a patient interface to the face of the subject.

According to the fifth aspect, there is provided a computer program product, comprising instructions which, when executed by a processor, cause the computer-implemented method according to the fourth aspect to be carried out.

In a sixth aspect of the invention, there is provided a device comprising a processor adapted to execute the computer program product of the fifth aspect.

### BRIEF DESCRIPTION OF THE DRAWINGS

Exemplary embodiments will now be described, by way of example only, with reference to the following Figures, in which:
FIG. 1 depicts a respiratory support device according to a first embodiment of the invention;
FIG. 2 depicts a detailed view of the processor of the respiratory support device according to the first embodiment;
FIG. 3 depicts the computer-implemented method executed by the processor according to the first embodiment;
FIG. 4 depicts an example of use data;
FIG. 5 depicts the processor according to a second embodiment of the invention;
FIG. 6 depicts a representation of the output signal according to a third embodiment of the invention;
FIG. 7 depicts a device according to a fourth embodiment;
FIG. 8 depicts a computer-implemented method according to a fifth embodiment.

### DETAILED DESCRIPTION OF EMBODIMENTS

It should be understood that the detailed description and specific examples, while indicating exemplary embodiments of the devices and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

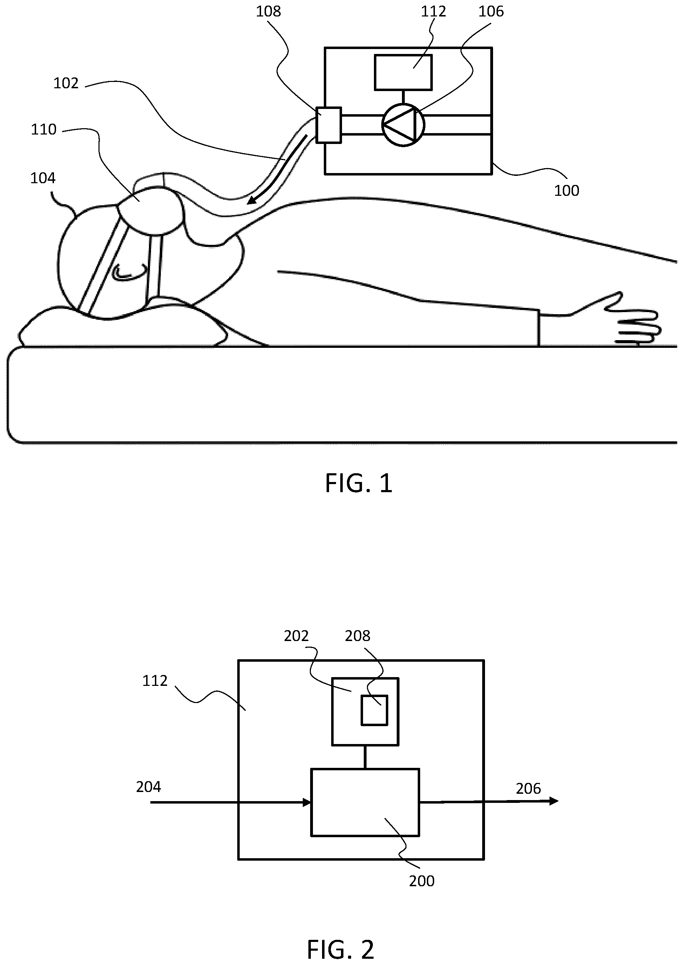

FIG. 1 depicts a respiratory support device 100 according to a first embodiment of the invention. The respiratory support device 100 is adapted to provide a pressurized airflow 102 to a subject 104. The respiratory support device 100 comprises an air pressure source 106, and an outlet 108. The air pressure source 106 is adapted to generate the pressurized airflow 102. The outlet 108 is connectable to a patient interface 110 to provide the pressurized airflow 102 to the subject 104. The respiratory support device 100 comprises a processor 112 configured to receive use data 204 representative of use of a respiratory support device 100 by a subject 104.

For example, the use data 204 is based on when the air pressure source 106 starts and stops. For example, the use data 204 is based on when the respiratory support device 100 is switched on and when the respiratory support device 100 is switched off. For example, the use data 204 is based on a sensor data from the respiratory support device 100. The sensor data is representative of a pressure and/or a flow rate of the pressurized airflow 102. The processor 112 is configured to determine when the patient interface 110 provides the pressurized airflow 102 to the subject's airway based on the sensor data. For example, the sensor data includes data representative of whether the subject is asleep. Such sensor data includes, for example, breathing data or cardiac data. For example, the use data 204 is based on the time the subject 104 is asleep and the patient interface 110 provides the pressurized airflow 102 to the subject's airway.

FIG. 2 depicts a detailed view of the processor 112 of the respiratory support device 100 according to the first embodiment. The processor 112 comprises a processor unit 200 and a memory 202. The processor unit 200 is configured to receive the use data 204. The memory 202 comprises a computer program product. The processor unit 200 and the memory 202 are configured to communicate with each other. The processor unit 200 is configured to execute the computer program product to cause the following computer-implemented method to be carried out. The processor 112 is configured to generate an output signal 206. The memory 202 stores a machine learning model 208.

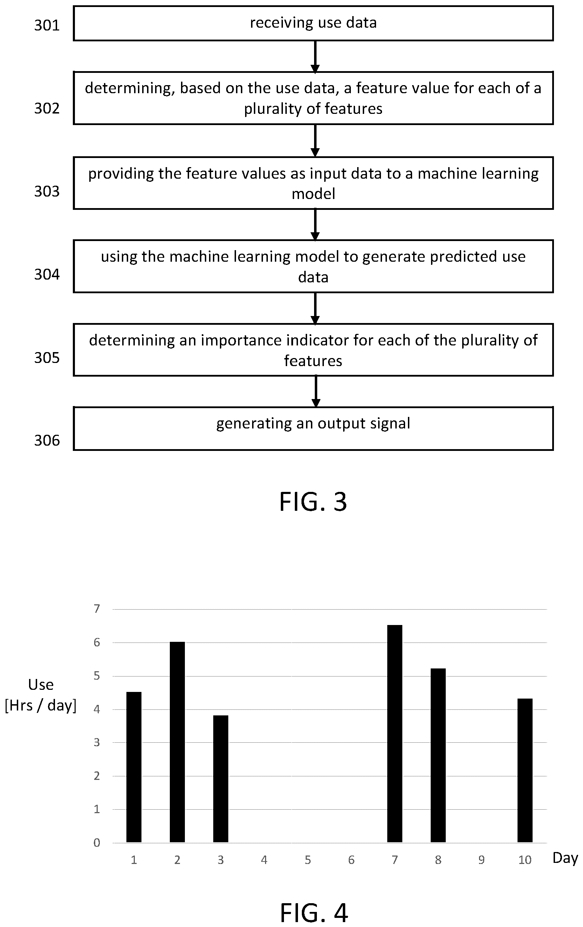

FIG. 3 depicts the computer-implemented method executed by the processor 112 according to the first embodiment. The computer-implemented method comprises, at 301, receiving use data 204 representative of use of a respiratory support device 100 by a subject 104. The respiratory support device 100 is adapted to provide the pressurized airflow 102 to the subject 104 during sleep. The computer-implemented method comprises, at 302, determining, based on the use data 204, a feature value for each of a plurality of features. The plurality of features are human-interpretable features representative of use of the respiratory support device 100. The computer-implemented method comprises, at 303, providing the feature values as input data to a machine learning model 208. The machine learning model 208 is trained to generate predicted use data 204 based on the feature values. The computer-implemented method comprises, at 304, using the machine learning model 208 to generate predicted use data 204 representative of a predicted use of the respiratory support device 100 by the subject 104. The computer-implemented method comprises, at 305, determining an importance indicator for each of the plurality of features. The importance indicators are representative of a contribution of the corresponding feature to the predicted use. The computer-implemented method comprises, at 306, generating the output signal 206 based on the importance indicators and the predicted use data 204. The output signal 206 is representative of a likelihood the subject 104 will stop using the respiratory support device 100.

For example, the processor 112 is configured to generate the output signal 206 including an alarm signal for alarming a care professional, in case the likelihood is above a critical threshold. The alarm signal prompts the care professional to take immediate action to prevent the subject 104 stops using the respiratory support device 100.

FIG. 4 depicts an example of use data 204. The graph in FIG. 4 depicts how many hours per day the subject 104 used the respiratory support device 100 for days 1-10. The subject 104 used the respiratory support device 100 on days 1-3 for respectively about 4, 6, and 4 hours. On days 4-6, the subject 104 did not use the respiratory support device 100. The subject 104 used the respiratory support device 100 on days 7 and 8 for respectively about 6.5, and 5 hours. On day 9, the subject 104 did not use the respiratory support device 100. The subject 104 used the respiratory support device 100 on day 10 for about 4 hours.

The processor 112 is configured to receive the use data 204. For example, the processor 112 is configured to receive a start time of use, and a stop time of use for each day. Based on the start times and stop times of use, the processor 112 is configured to calculate the duration of use for each day. In another example, the respiratory support device 100 provides data representative of the duration of use for each day. The use data 204 includes the number of nights of use, which is 6 nights of use in 10 days. The use data 204 includes the number of nights without use, which is 4 nights without use in 10 days. The use data 204 includes the number of consecutive nights without use. The maximum number of consecutive nights without use is 3 nights in 10 days.

Based on the use data 204, the processor 112 is configured to determine feature values of the plurality of features. For example, a first feature is a mean use of the respiratory support system over the 10 days. The mean is a statistical parameter based on the use data 204. The mean is the sum of the use of all days divided by the number of days. A second feature is the number of nights without use. A third feature is the number of consecutive nights without use. Based on the use data 204, the processor 112 determines that the first feature value is 3.0. Based on the use data 204, the processor 112 determines that the second feature value is 4. Based on the use data 204, the processor 112 determines that the third feature value is 3. The processor 112 provides the first feature value, the second feature value and the third feature value as input data to the machine learning model 208. The machine learning model 208 generates the predicted use data 204 based on the first feature value, the second feature value, and the third feature value. The processor 112 determines the importance indicators for each of the first feature, the second feature, and the third feature. Based on the importance indicators and the predicted use data 204, the processor 112 generates the output signal 206.

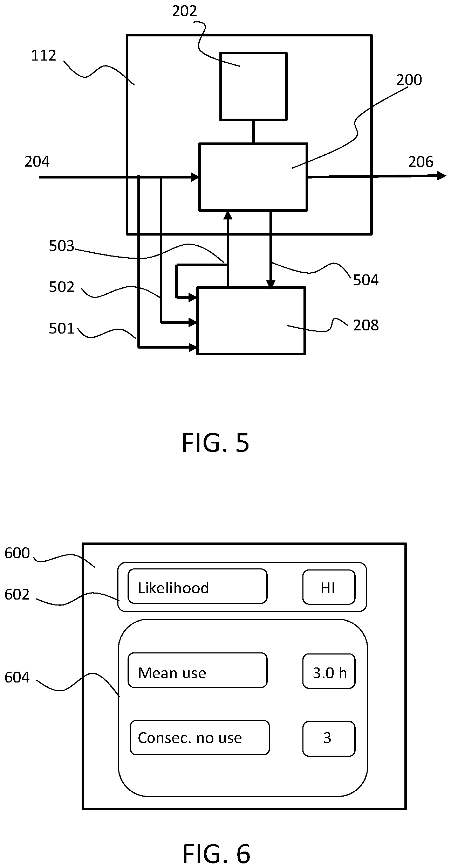

FIG. 5 depicts the processor 112 according to a second embodiment of the invention. The second embodiment has, for example, the same elements as the first embodiment, except for the following.

In the second embodiment, the processor 112 is configured to perform the computer-implemented method comprising retraining the machine learning model 208 to learn a relation between input retraining data 501 and output retraining data 502. The input retraining data 501 comprises a first portion of the use data 204 corresponding to a first use time period. The computer-implemented method comprises using as output retraining data 502 a second portion of the use data 204 corresponding to a second use time period. The second use time period is after the first use time period.

As depicted in FIG. 5, the processor 112 receives the use data 204. The processing unit 200 provides a signal 504 representative of the use data 204 to the machine learning model 208. In response to the signal 504, the machine learning model 208 generates output data 503, and provides the output data 503 to the processing unit 200. Based on the output data 503, the processing unit 200 generates the predicted use data 204.

The processor 112 is further configured to provide use data 204 as input retraining data 501 and output retraining data 502 to the machine learning model 208. In response to the input retraining data 501, the machine learning model 208 generates output data 503. The machine learning model 208 is retrained to learn the relation between the input retraining data 501 and the output retraining data 502. For example, the machine learning model 208 is updated or fine-tuned based on the relation between the input retraining data 501 and the output retraining data 502. For example, the retraining of the machine learning model 208 takes the output data 503 into account.

FIG. 5 depicts the machine learning model 208 as being external to the processor 112. For example, the machine learning model 208 is on a server or in the cloud or on a device external to the processor 112. The processor 112 is configured to communicate with the machine learning model 208. In another embodiment, the machine learning model 208 is stored in the memory 202 of the processor 112. In another embodiment, the machine learning model 208 is partly stored in the memory 202 of the processor 112 and is partly external to the processor 112.

For example, the first use time period is at least 90 days.

For example, when the computer-implemented method uses the machine learning model 208, this comprises using an extreme gradient boosting (XGB) algorithm. For example, the machine learning model 208 is or comprises an XGB-algorithm.

FIG. 6 depicts a representation of the output signal 206 according to a third embodiment of the invention. The third embodiment has, for example, the same features as the first embodiment, or as the second embodiment, except for the following.

In the third embodiment, the computer-implemented method comprises comparing the importance indicators with at least one threshold. The threshold is representative of the importance indicator having at least a significant contribution to the predicted use. The computer-implemented method comprises determining a subset of importance indicators exceeding the at least one threshold. The computer-implemented method comprises generating the output signal 206 based on the subset of importance indicators.

FIG. 6 depicts an image 600 as provided by a display based on the output signal 206. To illustrate the third embodiment, FIG. 6 makes use of the features values as used to illustrate the first embodiment. The output signal 206 represents that the likelihood 602 of the subject 104 stopping use of the respiratory support device 100 is high. The processor 112 determines that the first feature value and the third feature value have a significant contribution to the predicted use. The processor 112 determines that the second feature value does not have a significant contribution to the predicted use. The processor 112 generates the output signal 206 to represent the first feature and the third feature, but not the second feature. The processor 112 generates the output signal 206 to represent the first feature value and the third feature value, but not the second feature value. The first feature is the mean use. The third feature is the number of consecutive nights without use. The image 600 displays the subset 604 of the importance indicators exceeding the at least one threshold. Based on the likelihood, the care professional is alerted that the subject 104 has a high likelihood of stopping use. Based on the first feature and the third feature, the care professional is able to intervene to prevent the subject 104 from stopping use. The first feature and the third feature are ranked. The first feature contributes more to the predicted use than the third feature, so the first feature is placed at the top of the list above the third feature.

In an embodiment, the computer-implemented method comprises determining an odds ratio for at least one importance indicator. The odds ratio is representative of an association between the importance indicator and the predicted use. The odds ratio exceeds a threshold when the odds ratio is outside a range of 90%-110%. Using the example as displayed in FIG. 6, the odds ratio for the importance indicator of the first feature is less than 90%, the odds ratio for the importance indicator of the second feature is between 90% and 110%, and the odds ratio for the importance indicator of the third feature is more than 110%.

In an embodiment, the output signal 206 is indicative of a predicted time the subject 104 continues using the respiratory support device 100. For example, the output signal 206 causes the display of FIG. 6 to indicate a number of days the subject 104 is predicted to keep on using the respiratory support device 100. In an embodiment, the output signal 206 is indicative of a predicted level of use at which the subject 104 continues using the respiratory support device 100. For example, the output signal 206 causes the display of FIG. 6 to indicate a number of hours per night that the subject 104 is predicted to keep on using the respiratory support device 100.

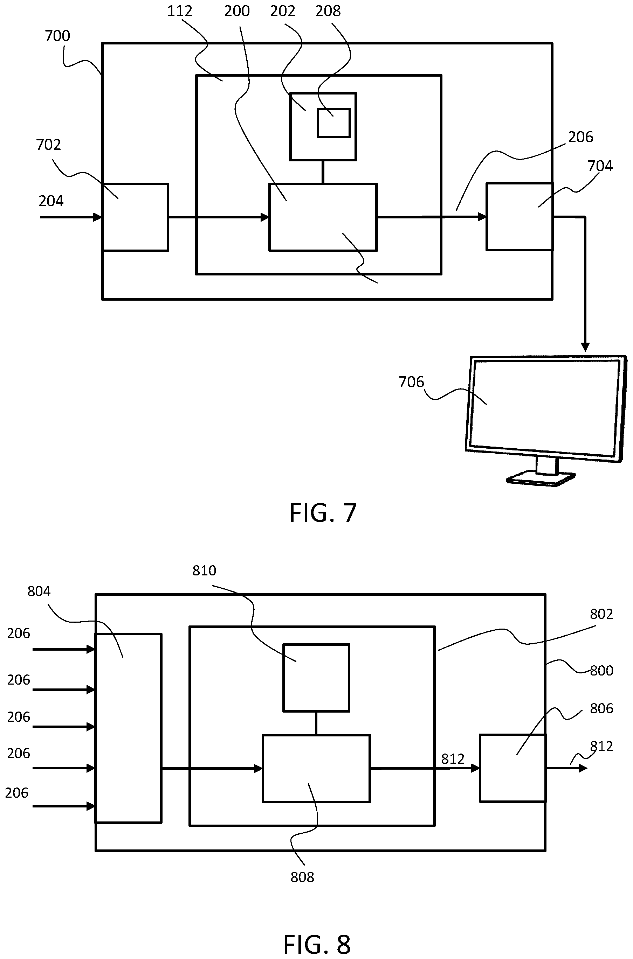

The processor 112 is adapted to execute the computer program product as described above to cause the computer-implemented method to be carried out. The processor 112 is, for example, arranged in the respiratory support device 100. In another embodiment, as depicted in FIG. 7, the processor 112 is arranged in a device 700 different than the respiratory support device 100.

FIG. 7 depicts a device 700 according to a fourth embodiment. The fourth embodiment has, for example, the same elements as the first embodiment, the second embodiment, or the third embodiment, except for the following. The device 700 is different from the respiratory support device 100. The device 700 has a data receiver 702 and a signal output 704. The data receiver 702 is adapted to communicate with the respiratory support device 100 to receive the use data 204 from the respiratory support device 100. The processor 112 is adapted to receive the use data 204 from the data receiver 702.

The signal output 704 is adapted to communicate with a display 706 device to provide the output signal 206 to the display 706. For example, the data receiver 702 is adapted to receive the use data 204 via Bluetooth, or Wi-Fi, or WLAN, or any other type of telecommunication. For example, the signal output 704 is adapted to send the output signal 206 via Bluetooth, or Wi-Fi, or WLAN, or any other type of telecommunication.

FIG. 8 depicts a computer-implemented method according to a fifth embodiment. The computer-implemented method according to the fifth embodiment is referred to as the further computer-implemented method. The further computer-implemented method comprises receiving for a plurality of subjects a plurality of output signals 206 generated by the computer-implemented method according to any one of the first embodiment, the second embodiment, the third embodiment, or the fourth embodiment. The further computer-implemented method comprises classifying the plurality of subjects to a plurality of classes based on the importance indicators and the likelihood. The further computer-implemented method comprises selecting at least one of the plurality of classes as a candidate class for intervention. The further computer-implemented method comprises generating a class output signal 812 indicative of the importance indicators and the likelihood of the class for the candidate class for intervention.

FIG. 8 depicts a further device 800. The further device has a further processor 802, a further signal receiver 804, and a class signal output 806. The further processor 802 comprises a further processing unit 808 and a further memory 810. The further device 800 receives the output signals 206 from multiple subjects via the further signal receiver 804. The further processor 802 unit is configured to receive the output signals 206 from the further signal receiver 804. The further memory 810 comprises a further computer program product. The further processor unit 808 and the further memory 202 are configured to communicate with each other. The further processor unit 808 is configured to execute the further computer program product to cause the further computer-implemented method to be carried out. Based on the output signals 206, the processor 802 classifies the plurality of subjects to a plurality of classes according to the importance indicators and the likelihood. The further processor 802 selects at least one of the plurality of classes as a candidate class for intervention. The further processor 802 generates a class output signal 812 indicative of the importance indicators and the likelihood of the class for the candidate class for intervention. The class signal output 806 sends the class output signal 812, for example, to another device or to a database or to a display.

It will be understood that the disclosed methods are computer-implemented methods. As such, there is also proposed a concept of a computer program comprising code means for implementing any described method when said program is run on a processor.

The skilled person would be readily capable of developing the processor 112 or the further processor 802 for carrying out the computer-implemented method or the further computer-implemented method. Thus, each step of a flow chart may represent a different action performed by a processor, and may be performed by a respective module of the processor.

As discussed above, the devices 100, 700, and 800 make use of a processor to perform the data processing. The processors 112 and 802 can be implemented in numerous ways, with software and/or hardware, to perform the various functions required. The processor may employ one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). Thus, the processor may be embodied as a digital and/or analog processing system.

In various implementations, the memories 202 and 810 may comprise storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processor or may be transportable, such that the one or more programs stored thereon can be loaded into a processor.

Functions implemented by a processor 112 or processor 802 may be implemented by a single processor or by multiple separate processing units which may together be considered to constitute a "processor ". Such processing units may in some cases be remote from each other and communicate with each other in a wired or wireless manner.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Variations to the disclosed embodiments can be understood and effected by those skilled in the art in practicing the principles and techniques described herein, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A computer-implemented method comprising:
receiving use data (204) representative of use of a respiratory support device (100) by a subject (104),
wherein the respiratory support device (100) is adapted to provide a pressurized airflow (102) to the subject (104) during sleep;
determining, based on the use data (204), a feature value for each of a plurality of features,
wherein the plurality of features are human-interpretable features representative of use of the respiratory support device (100),
providing the feature values as input data to a machine learning model (208),
wherein the machine learning model (208) is trained to generate predicted use data (204) based on the feature values;
using the machine learning model (208) to generate predicted use data (204) representative of a predicted use of the respiratory support device (100) by the subject (104);
determining an importance indicator for each of the plurality of features,
wherein the importance indicators are representative of a contribution of the corresponding feature to the predicted use;
generating an output signal (206) based on the importance indicators and the predicted use data (204),
wherein the output signal (206) is representative of a likelihood the subject (104) will stop using the respiratory support device (100).

2. The computer-implemented method according to claim 1, wherein the use data (204) comprises at least one of a duration of use, a start time of use, a stop time of use, a number of nights of use, a number of nights without use, or a number of consecutive nights without use.

3. The computer-implemented method according to any of the preceding claims, wherein the plurality of features comprises at least one of a mean use of the respiratory support system per night, a number of nights without use, and a number of consecutive nights without use.

4. The computer-implemented method according to any one of preceding claims, wherein the plurality of features comprises a statistical parameter based on the use data (204).

5. The computer-implemented method according to any one of the preceding claims, comprising:
retraining the machine learning model (208) to learn a relation between input retraining data (501) and output retraining data (502),
wherein the input retraining data (501) comprises a first portion of the use data (204) corresponding to a first use time period;
using as output retraining data (502) a second portion of the use data (204) corresponding to a second use time period,
wherein the second use time period is after the first use time period.

6. The computer-implemented method according to claim 5, wherein the first use time period is at least 90 days.

7. The computer-implemented method according to any one of the preceding claims, wherein using the machine learning model (208) comprises using an extreme gradient boosting (XGB) algorithm.

8. The computer-implemented method according to any one of the preceding claims, comprising:
comparing the importance indicators with at least one threshold,
wherein the threshold is representative of the importance indicator having at least a significant contribution to the predicted use;
determining a subset of importance indicators exceeding the at least one threshold;
generating the output signal (206) based on the subset of importance indicators.

9. The computer-implemented method according to claim 8, comprising determining an odds ratio for at least one importance indicator,
wherein the odds ratio is representative of an association between the importance indicator and the predicted use,
wherein the odds ratio exceeds the at least one threshold when the odds ratio is outside a range of 90%-110%.

10. The computer-implemented method according to any one of the preceding claims, comprising
generating the output signal (206) to include an alarm signal for alarming a care professional, in case the likelihood is above a critical threshold.

11. The computer-implemented method according to any one of the preceding claims, wherein the output signal (206) is indicative of one or more of i) a predicted time the subject (104) continues using the respiratory support device (100), or ii) a predicted level of use at which the subject (104) continues using the respiratory support device (100).

12. A computer program product, comprising instructions which, when executed by a processor (112) cause the computer-implemented method according to any one of the preceding claims to be carried out.

13. A device comprising a processor (112) adapted to execute the computer program product of claim 12.

14. The device according to claim 13, comprising a data receiver and a signal output,
wherein the data receiver is adapted to communicate with the respiratory support device (100) to receive the use data (204) from the respiratory support device (100),
wherein the processor (112) is adapted to receive the use data (204) from the data receiver,
wherein the signal output is adapted to communicate with a display device to provide the output signal (206) to the display device.

15. A computer-implemented method, comprising:
receiving for a plurality of subjects a plurality of output signals generated by the method according to any one of claims 1-11;
classifying the plurality of subjects to a plurality of classes based on the importance indicators and the likelihood;
selecting at least one of the plurality of classes as a candidate class for intervention;
generating a class output signal (206) indicative of the importance indicators and the likelihood of the class for the candidate class for intervention.
